# EUROPEAN PATENT SPECIFICATION

(11) **EP 1 422 058 B1**
(45) Date of publication and mention of the grant of the patent: **24.11.2010**
(21) Application number: 03256663.0
(22) Date of filing: 22.10.2003
(51) Int. Cl.: B32B 27/30, B32B 27/32, B32B 25/16, A61L 15/22, A61L 15/24, A61K 9/70, A61F 13/02

(54) **Adhesive bandage having an improved backing material**
Klebender Wundverband mit verbessertem Trägermaterial
Pansement adhésif ayant un support amelioré

(30) Priority: 23.10.2002 US 279044
(43) Date of publication of application: 26.05.2004
(73) Proprietor: JOHNSON & JOHNSON CONSUMER COMPANIES, INC., Skillman, NJ 08558 (US)
(72) Inventor: Franca, Fabio Eduardo, Sao Jose dos Campos-SP, 12242-903 (BR); Patel, Shailesh C., IN 47802 (US); Aledo, Maria Aparecida de Carvalho Scamilla, Sao Jose Dos Campos - SP, 12242-040 (BR); Ray, Carl Douglas, Terre Haute, IN 47805 (US)
(74) Representative: Mercer, Christopher Paul

(56) References cited:
- EP-A- 0 713 764
- WO-A-02/20067
- PATENT ABSTRACTS OF JAPAN vol. 1998, no. 14, 31 December 1998 (1998-12-31) & JP 10 234838 A (IDEMITSU PETROCHEM CO LTD), 8 September 1998 (1998-09-08) & DATABASE WPI Derwent Publications Ltd., London, GB; AN 1998-535459

## Description

### BACKGROUND OF THE INVENTION

### Field of the Invention

The present invention relates to adhesive bandages for application to skin wounds, irritations, abrasions, bruises and the like. More specifically, this invention relates to adhesive bandages that include an improved backing material. The backing material is a flexible polymeric film made from two non-elastic polymer outer layers and an elastic polymer inner layer.

### Description of the Prior Art

For many years, adhesive bandages have been sold for the purpose of assisting in the treatment and/or protection of cuts, bruises, abrasions and similar injuries to the skin. These bandages cover and protect the wound while it heals. Such bandages comprise a backing material, one major surface of which is coated with an adhesive. A wound-contacting pad, which in use overlays the wound, is secured to the adhesive coated surface of the backing (usually, but not always, in the generally central region thereof) by a portion of the adhesive composition. The remaining portions of the adhesive composition serve, during use, to adhere the bandage to the skin surrounding the wound site.

Backing materials made from polymers such as polyethylene and polyvinylchloride are known in the art. Such backing materials have the desirable property of being liquid water impermeable. On the other hand, these backing materials are also impermeable to gases such as oxygen and water vapor. If water vapor cannot evaporate from the skin under a backing material, the skin tends to macerate and become uncomfortable. Therefore, backing materials which are both liquid water impermeable and water vapor impermeable are typically perforated to allow water vapor to evaporate from the skin, i.e., to allow the skin to "breathe".

For use on areas that bend, such as on fingers, backing materials that have good flexibility are particularly useful. The flexibility of a backing material is typically measured by stretching the material on an instrument such as an Instron. Two measurements of flexibility are important. The first measurement is the amount of force in grams necessary to stretch the backing material beyond its normal length. The test utilized for stretching backing materials is known as the "stress/strain" test.

Areas of the body that bend, such as finger joints, knees, and elbows were studied to determine how much the skin stretches when the joints are flexed. It was discovered that the skin over these areas stretches 50% or less. Therefore, it was concluded that backing materials for use in adhesive bandages that are intended for application to "flexing" or "bending" body parts should be tested, for example, for their permanent set and stress/strain characteristics, at 50% elongation.

The second measurement of flexibility is the amount of permanent deformity to the backing material after being stretched. This is known as the "permanent set" test.

Polyethylene film backings typically pass the stress/strain test, but do not pass the permanent set test and therefore are not ideal for use on areas that bend, as the bandage will not be comfortable. Polyvinyl chloride and polyurethane film backings typically pass both the stress/strain test and the permanent set test, and therefore have been ideal for use on areas that bend.

There are environmental concerns over residual monomers in polyvinyl chloride film manufacturing. Additionally, polyvinyl chloride backings are somewhat expensive. Therefore, there is a need for an adhesive bandage whose flexibility properties are similar to the flexibility properties of adhesive bandages made with polyvinyl chloride film backings, but which is less expensive and does not raise potential environmental concerns.

WO 02/20067 discloses a multi-layer wound dressing comprising at least two absorbent layers. The absorbent layers have different absorbencies, with the layer closest to the wound having a lower absorbency than the layer furthest from the wound. The wound dressing typically contains additional non-absorbing layers, such as a backing film and wound-facing film. The dressing provides absorbency of wound exudates while preventing the absorbent material from entering the wound.

JP 10234838 discloses a film for affixing base which is excellent in flexibility, elasticity and chemical resistance, which does not generate environmental problems. The film is of a two or three layer structure comprising: a first layer; a second layer, which comprises an ethylene-α-olefin copolymer; and optionally a third layer, wherein the first and third layers comprise a polyolefin-based polymer.

EP 0 713 764 discloses a film having at least three layers, matt on both sides and with a low tendency to rolling up, which is made by coextrusion. The outer layers are formed from a mixture of at least two different polymers and rather than being flexible, the films disclosed in this document are stiff/rigid.

United States Patent Number 6,326,081 teaches an improved masking film for use to protect various substrates including acrylics, glass, metals, and ceramics. The masking film is matte finished on one side and does not utilize an adhesive to adhere to the substrate. The film is coextruded and utilizes copolymers, comonomers, and mixtures thereof.

Despite the disclosure of the prior art, there is a continuing need for a bandage that has similar flexibility properties as bandages made with polyvinyl chloride film backings, but is less expensive and does not have toxicity concerns.

### SUMMARY OF THE INVENTION

In accordance with the present invention, an adhesive bandage as defined in claim 1 comprises a backing material; an adhesive applied to said backing material; and a wound-contacting pad applied to said adhesive; wherein said backing material comprises a first outer layer comprising a thermoplastic polymer; an inner layer comprising an elastic polymer; and a second outer layer comprising a thermoplastic polymer. The adhesive, preferably a skin-compatible, medically acceptable pressure-sensitive adhesive, is applied to and carried by one major surface of the backing material to adhere the wound contacting pad to the underlying portion of the backing film. As is known, the remaining portions of adhesive serve to affix the bandage to the skin during use.

### DETAILED DESCRIPTION OF THE DRAWINGS

The invention will be more clearly understood by reference to the accompanying drawings on which:
FIG. 1 is an exploded perspective, with some portions turned, of one embodiment of an adhesive bandage in accordance with the present invention;
FIG. 2 is a perspective view of the bandage of FIG. 1; and
FIG. 3 is an enlarged longitudinal cross-section taken along line 3-3 of FIG. 2.

### DETAILED DESCRIPTION OF PREFERRED EMBODIMENTS

Polymeric films useful as backing materials in the practice of the present invention are taught in United States Patent Number 6,326,081, the entire disclosure of which is hereby incorporated by reference. Suitable processes for making such polymeric films are also taught in the '081 patent.

The backing material includes first and second outer layers, each comprising a thermoplastic polymer. The first and second thermoplastic polymer layers may be made of the same polymers or different polymers. Suitable polymers for the outer layers include, but are not limited to, polyolefins such as low density polyethylene and linear low density polyethylene; polyvinyl alcohol; nylon; polyester; polystyrene; polymethylpentene; polyoxymethylene; copolymers thereof, and mixtures thereof. A mixture of low density polyethylene and linear low density polyethylene is preferred for use as the outer layers of the backing film in the practice of the present invention.

The backing material also includes an inner polymeric layer comprising an elastic polymer. Suitable elastic polymers for the inner layer include, but are not limited to, a metallocene catalyzed copolymer of ethylene with a comonomer selected from the group consisting of octene, hexene, and butene; ethylene propylene diene monomer; styrene copolymers, such as styrene-butadiene-styrene and styrene-ethylene-butylene-styrene copolymers and the like; ethylene methyl acrylate copolymer; ethylene vinyl acetate and mixtures thereof. A mixture of a metallocene catalyzed polyethylene copolymer, wherein the comonomer in the polyethylene copolymer is octene, and an ethylene methyl acrylate copolymer is preferred for use as the inner layer of the backing material in the practice of the present invention.

The backing materials of the present invention are typically made by co-extrusion; other methods may be used if desired. The basis weight for the backing materials may range from about 30 g/m² to about 120 g/m², preferably from about 50 g/m² to about 90 g/m². The thickness of the backing material may range from about 0.03 mm to about 3 mm, preferably from about 0.05 mm to about 0.5 mm.

The backing material preferably is embossed so that one side is smooth and the other side has a matte finish.

Suitable backing materials for use in the present invention have a permanent set of no more than about 15 percent. It has been found that adhesive bandages made from backing materials whose permanent set exceeds about 15 percent do not continue to conform to the body part to which they have been applied after that body part has been repeatedly flexed during the course of daily activities. Failure of the backing material to recover after it has been elongated tends to lead to "gapping" between the bandage and the body part to which it has been applied.. Suitable backing materials for use in the present invention also have a stress/strain relationship at 50 percent elongation of between about 800 g/25.4 mm sample width and 1500 g / 25.4 mm sample width.

As is known in the art, the wound-contacting pad of an adhesive bandage protects the wound from contamination by dirt. The absorbent pad may be made from various materials including rayon fibers; natural fibers, such as, but not limited to, cotton and wood pulp fibers, and synthetic fibers, such as, but not limited to, polyester, polyamide, and polyolefin fibers. Synthetic fibers comprising two or more polymers may be used. Blends of fibers may be used. The fibers may be bicomponent fibers. For example, the fibers may have a core of one polymer, and a sheath of a different polymer. The denier of the fibers comprising the wound-contacting pad is not limited, but typically ranges from about 1 to 10 denier.

The basis weight of the wound-contacting pad is not limited, but typically ranges from 0.003 g/cm² to 0.015 g/cm². The size of the wound-contacting pad may vary depending on the size of the bandage and/or the size of the wound to be protected or treated.

Typically, an adhesive is used to adhere the wound contacting pad to the backing material and to adhere the adhesive bandage to the skin of the user. The adhesive may be an aqueous or solvent-based adhesive or it may be a hot melt adhesive, as desired. Examples of suitable adhesives include, but are not limited to, those based on styrenic block copolymers and tackifying resins such as HL-1491 available from HB-Fuller Co. (St. Paul MN), H-2543 available from ATO-Findley (Wawatausa, WI), and Resyn 34-5534 available from National Starch & Chemical Company (Bridgewater, NJ). Ethylene copolymers, including ethylene vinyl acetate copolymers, are also useful as adhesives.

Suitable adhesives also include acrylic based, dextrin based, and urethane based adhesives as well as natural and synthetic elastomers. The adhesives may also include amorphous polyolefins including amorphous polypropylene, such as HL-1308 available from HB Fuller or Rextac RT 2373 available from Huntsman (Odesssa, TX). The adhesive may be based on Kraton® Brand synthetic elastomers, or natural rubber. These adhesives may also include tackifiers, anti-oxidants, processing oils, and the like as is known in the art.

The adhesive can be applied in any desired manner, e.g., by spraying, screen printing or slot die coating. The amount of adhesive typically applied is well known in the art. Generally, the adhesive coating weight varies from about 20 grams per square meter ("gsm") to about 100 gsm.

Bandages in accordance with the invention may be square, rectangular, round, oval, or triangular in shape. The size of the bandage will depend on the shape of the bandage and the size of the wound meant to be covered by the bandage. Generally, a square bandage may range in size from 2 cm x 2 cm to 15 cm x 15 cm. The length of a rectangular bandage may range from 5 cm to 15 cm, preferably from 7.5 cm to 12.5 cm. The width of a rectangular bandage may range from 0.5 cm to 5 cm, preferably from 1 cm to 3 cm.

The thickness of the bandage of the invention will vary depending on the application, but generally may range from 0.25 mm to 5 mm, preferably I mm to 3 mm, more preferably 1 mm to 2 mm.

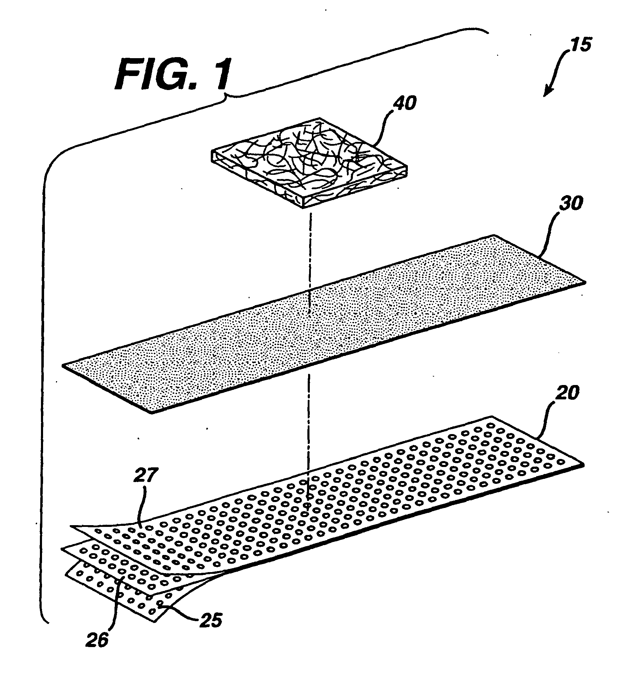

Referring now to the accompanying drawings, FIGS. 1-3 thereof illustrate one embodiment of an adhesive bandage in accordance with the present invention. Adhesive bandage 15 comprises a backing material 20, adhesive 30, and a wound-contacting pad 40. If desired, a wound release layer, e.g., a porous polyethylene netting, may be secured to that surface of wound-contacting pad 40 which will face toward the wound when the bandage is used. This wound release layer prevents undesirable adherence of the wound-contacting pad to the underlying wound site. The wound contacting pad 40 and the exposed portions of adhesive adjacent thereto may be covered with protective release sheets (not shown in the drawings) prior to packaging of the bandage.

In the embodiment of FIGS. 1-3, backing material 20 has a thickness of about 0.0762 mm (3 mils) and has a first major surface 22 and a second major surface 23. Backing material 20 comprises first outer polymeric layer 25 and second outer polymeric layer 27. These outer layers are made from a 50:50 mixture (by weight) of low density polyethylene (CHEVRON PHILLIPS LDPE 1017 coating resin) and linear low density polyethylene (Dowlex 2517 from DOW CHEMICAL). Backing material 20 further comprises an inner elastic polymeric layer 26, coextensive with outer layers 25, 27. Polymeric layer 26 is made of a mixture of 58% by weight metallocene catalyzed ultra low density polyethylene copolymer (AFFINITY® PL 1280 from DOW CHEMICAL) and 42% by weight ethylene methyl acrylate copolymer (TC 120 from EXXON CHEMICAL COMPANY). The thickness of backing 20 may range from 0.03 mm to about 3 mm, preferably 0.05 mm to 0.5 mm, even more preferably 0.07 mm to 0.15 mm.

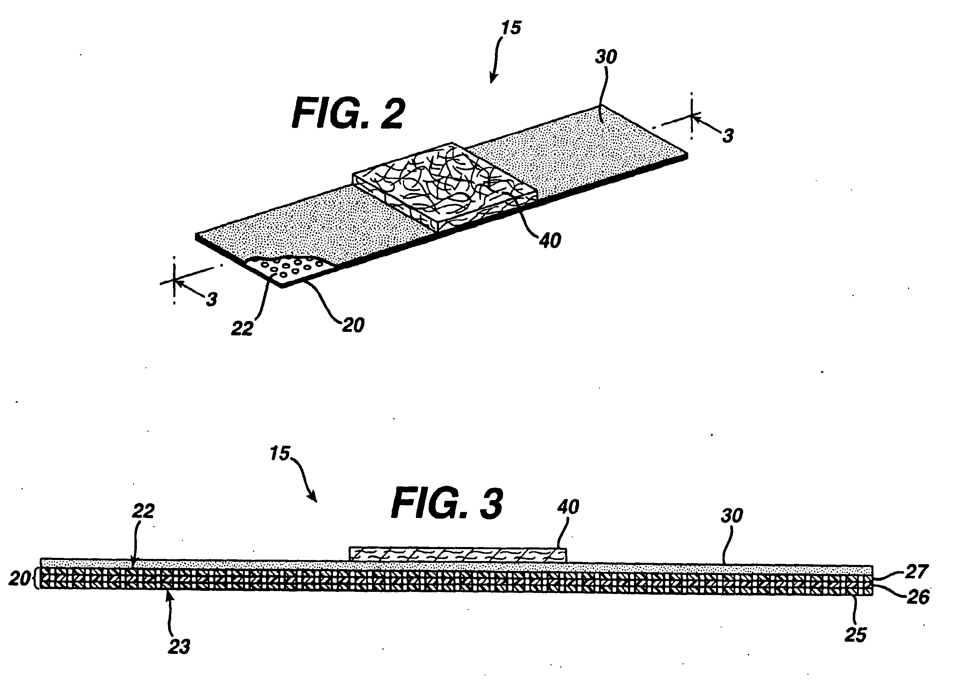

The first major surface 22 of the backing material carries a pressure sensitive adhesive. The adhesive is indicated by the stippling on first major surface 22 of backing material 20 in FIGS. 1 and 2 and by numeral 30 in FIG. 3. This adhesive is applied by slot die coating to the aforementioned backing material at a level of about 50 grams of adhesive per square meter of backing material 50 gsm.

Wound-contacting pad 40, which comprises a blend of 10% by weight rayon fibers and 90% polypropylene fibers, is secured to tri-layer backing film 20 by adhesive 30. The basis weight of this wound-contacting pad 40 is 115 g/m² (3.7 ounces/sq. yard). Physical integrity of wound-contacting pad 40 is achieved by the frictional engagement of the fibers or blend of fibers of which it is constituted. Physical integrity of the wound-contacting pad may be enhanced, if necessary or desired, by the application thereto of a binder as is known in the art.

Although not a necessary feature of the present invention, it is preferable to cover the upper surface of wound-contacting pad 40 with a wound release means. As is known in the art, an apertured plastic film or netting, e.g., one made from polyethylene or the like, may serve as such wound release means. Apertured plastic films suitable for covering the wound-contacting pad are commercially available, e.g., from Applied Extrusion Technology, Middletown, Delaware 19709 USA.

Following are the dimensions of the structural components of one typical adhesive bandage of the type shown in FIGS. 1-3: length of backing material 20 = 7.5 cm; width of backing material 20 = 2.5 cm; length of wound-contacting pad 40 = 1.8 cm; width of wound-contacting pad 40 = 1.8 cm.

### Preparation of Bandages

Bandages according to the present invention can be made as follows: A strip of backing material having the desired dimensions is placed on a work surface and the selected adhesive is applied to one major surface thereof. The wound-contacting pad is then secured to the adhesive. The wound-contacting pad may be centered end-to-end of the backing or it may be offset toward one such end, as may be desired. The wound release layer, if it is to be used, is secured to the upper surface of the wound-contacting pad. Release strips, e.g., siliconized paper, are placed over the exposed portions of adhesive as well as the wound-contacting pad to protect the bandage prior to use. The bandage is then packaged in any convenient manner, for example by enclosing it between two layers of heat sealable paper and heat sealing the periphery of the two layers. The packaged bandage is then sterilized, if desired, by techniques well known in the art. The bandages can be made by hand or on commercially available bandage making equipment.

The following Example is provided to further illustrate the adhesive bandages of the present invention.

### Example 1

The following backing materials were prepared by co-extrusion:
Backing 1: First outer polymeric layer - a blend of 50% by weight low density polyethylene (CHEVRON PHILLIPS LDPE 1017 coating resin) and 50% by weight linear low density polyethylene (Dowlex 2517 from DOW CHEMICAL); inner elastic polymeric layer - a blend of 58% by weight metallocene catalyzed ultra low density polyethylene copolymer (AFFINITY® PL 1280 from DOW CHEMICAL) and 42% by weight ethylene methyl acrylate copolymer (OPTEMA® TC 120 from EXXON CHEMICAL); second outer polymeric layer - a blend of 50% by weight low density polyethylene (CHEVRON PHILLIPS LDPE 1017 coating resin) and 50% by weight linear low density polyethylene (Dowlex 2517). The basis weight of backing material 1 was 70 g/m². The comonomer in the aforementioned metallocene catalyzed ultra low density polyethylene copolymer is octene.
Backing 2: First outer polymeric layer - a blend of 50% by weight low density polyethylene (CHEVRON PHILLIPS LDPE 1017 coating resin) and 50% by weight linear low density polyethylene (Dowlex 2517); inner polymeric elastic layer - a blend of 5.1% by weight fleshtone color concentrate (AMPACET CORPORATION), 52.9% by weight metallocene catalyzed ultra low density polyethylene copolymer (PL 1280 from DOW CHEMICAL) and 42% by weight ethylene methyl acrylate copolymer (OPTEMA® TC 120 from EXXON CHEMICAL); second outer polymeric layer - a blend of 50% by weight low density polyethylene (CHEVRON PHILLIPS LDPE 1017 coating resin) and 50% by weight linear low density polyethylene (Dowlex 2517). The basis weight of backing material 2 was 70 g/m².
Backing 3: First outer polymeric layer - a blend of 50% by weight low density polyethylene (CHEVRON PHILLIPS LDPE 1017 coating resin) and 50% by weight linear low density polyethylene (Dowlex 2517); inner elastic polymeric layer - a blend of 6% by weight white concentrate (AMPACET CORPORATION-70% white pigment in ethylene methyl acrylate), 55% by weight metallocene catalyzed polyethylene copolymer (PL 1280 from DOW CHEMICAL) and 39% by weight ethylene methyl acrylate copolymer (TC 120 from EXXON CHEMICAL); second outer polymeric layer - a blend of 50% by weight low density polyethylene (CHEVRON PHILLIPS LDPE 1017 coating resin) and 50% by weight linear low density polyethylene (Dowlex 2517). The basis weight of backing material 3 was 70 g/m².
Backings 1, 2 and 3 were tested in a Universal Testing Machine (Emic Company). Samples having a width of 25.4 mm and a length of about 80 mm were prepared for testing. The sample under test was clamped in the sample grips of the test machine so it was free of slack but was not under tension. The jaw speed was set at 127 mm/min throughout the test procedure. The initial distance between the opposed sample grips was set at 50 mm. The first cycle was started and the sample grips were extended so that the distance between them was 75 mm, i.e., the sample under test was elongated by 50%. After waiting 60 seconds at the elongated stage, the sample grips were returned to their initial separation of 50 mm. After a 30 second waiting period, the next cycle was begun. The permanent set was defined as the distance (i.e., the distance between the opposed sample grips) at which the force to elongate the material during the cycle exceeded zero divided by the initial distance between grips, i.e., 50 mm. The cycle was repeated 4 times (a total of 5 cycles) and the permanent set was recorded during cycles 2, 3, 4 and 5. The results of permanent set, in percent, during the fifth cycle for each sample are shown in Table 1. Stress values at 50 percent elongation are also shown in Table 1.

**Table 1**

| Sample | Permanent Set (%) | Stress (@, 50% Elongation (g/25.4 mm sample width) |
|---|---|---|
| **1** | **11** | **1172** |
| **2** | **11** | **1052** |
| **3** | **11** | **1024** |

The data above demonstrates that the backings of the present invention have desirable properties for an adhesive bandage. The adhesive bandages of the present invention are comfortable when worn over areas that bend, such as on fingers.

## Claims

1. An adhesive bandage comprising:
a backing material;
an adhesive applied to said backing material; and
a wound-contacting pad applied to said adhesive,
wherein:
said backing material comprises a first outer layer comprising a thermoplastic polymer; an inner layer comprising an elastic polymer; and a second outer layer comprising a thermoplastic polymer,
the backing has a permanent set of no more than 15 percent at 50% elongation; and
the backing has a stress at 50% elongation of between 800 g / 25.4 mm width and 1500 g / 25.4 mm width.

2. The adhesive bandage of claim 1 wherein the first outer layer and the second outer layer comprise the same thermoplastic polymer.

3. The adhesive bandage of claim 1 wherein the first outer layer and the second outer layer comprise different thermoplastic polymers.

4. The adhesive bandage of claim 1 wherein the first outer layer and the second outer layer comprise thermoplastic polymers selected from the group consisting of low density polyethylene, linear low density polyethylene, polyvinyl alcohol, nylon, polyester, polystyrene, polymethylpentene, polyoxymethylene, copolymers thereof, and mixtures thereof.

5. The adhesive bandage of claim 1 wherein the inner layer comprises an elastic polymer selected from the group consisting of metallocene catalyzed copolymer of ethylene with a comonomer selected from the group consisting of octene, hexene, and butene; ethylene propylene diene monomer; styrene copolymers; ethylene methyl acrylate copolymer; ethylene vinyl acetate copolymers and mixtures thereof.

6. The adhesive bandage of claim 1 wherein the outer layers comprise a mixture of low density polyethylene and linear low density polyethylene.

7. The adhesive bandage of claim 6 wherein the inner layer comprises a mixture of a metallocene catalyzed polyethylene copolymer and an ethylene methyl acrylate copolymer.

## Patentansprüche

1. Klebender Wundverband, umfassend:
ein Trägermaterial;
einen auf das Trägermaterial aufgebrachten Kleber; und
ein auf den Kleber aufgebrachtes Wundkontaktkissen,
wobei:
das Trägermaterial eine erste äußere Schicht, die ein thermoplastisches Polymer umfasst; eine innere Schicht, die ein elastisches Polymer umfasst; und eine zweite äußere Schicht umfasst, die ein thermoplastisches Polymer umfasst,
der Träger einen Verformungswert von nicht mehr als 15 Prozent bei 50% Verlängerung aufweist; und
der Träger eine Spannung bei 50% Verlängerung von zwischen 800 g / 25,4 mm Breite und 1500 g / 25,4 mm Breite aufweist.

2. Klebender Wundverband nach Anspruch 1, wobei die erste äußere Schicht und die zweite äußere Schicht dasselbe thermoplastische Polymer umfassen.

3. Klebender Wundverband nach Anspruch 1, wobei die erste äußere Schicht und die zweite äußere Schicht unterschiedliche thermoplastische Polymere umfassen.

4. Klebender Wundverband nach Anspruch 1, wobei die erste äußere Schicht und die zweite äußere Schicht thermoplastische Polymere umfassen, die ausgewählt sind aus der Gruppe, bestehend aus Low-Density-Polyethylen, linearem Low-Density-Polyethylen, Polyvinylalkohol, Nylon, Polyester, Polystyrol, Polymethylpenten, Polyoxymethylen, Copolymeren davon und Mischungen davon.

5. Klebender Wundverband nach Anspruch 1, wobei die innere Schicht ein elastisches Polymer umfasst, das ausgewählt ist aus der Gruppe, bestehend aus mit Metallocen katalysiertem Copolymer von Ethylen mit einem Comonomer, das ausgewählt ist aus der Gruppe, bestehend aus Octen, Hexen und Buten; Ethylen-Propylen-Dien-Monomer; Styrol-Copolymeren; Ethylen-Methylacrylat-Copolymer; EthylenVinylacetat-Copolymeren und Mischungen davon.

6. Klebender Wundverband nach Anspruch 1, wobei die äußeren Schichten eine Mischung aus Low-Density-Polyethylen und linearem Low-Density-Polyethylen umfassen.

7. Klebender Wundverband nach Anspruch 6, wobei die innere Schicht eine Mischung aus einem mit Metallocen katalysiertem Polyethylen-Copolymer und einem Ethylen-Methylacrylat-Copolymer umfasst.

## Revendications

1. Pansement adhésif qui comprend :
■ un support ;
■ un adhésif appliqué sur ledit support ; et
■ une compresse en contact avec la plaie appliquée sur ledit adhésif,
dans lequel :
ledit support comprend une première couche extérieure comprenant un polymère thermoplastique ; une couche intérieure comprenant un polymère élastique ; et une seconde couche extérieure comprenant un polymère thermoplastique,
le support ayant une déformation permanente de pas plus que 15 % à 50 % d'élongation ; et
le support ayant une contrainte à 50 % d'élongation comprise entre 800 g/25,4 mm de largeur et 1 500 g/25,4 mm de largeur.

2. Pansement adhésif selon la revendication 1, dans lequel la première couche extérieure et la seconde couche extérieure comprennent le même polymère thermoplastique.

3. Pansement adhésif selon la revendication 1, dans lequel la première couche extérieure et la seconde couche extérieure comprennent des polymères thermoplastiques différents.

4. Pansement adhésif selon la revendication 1, dans lequel la première couche extérieure et la seconde couche extérieure comprennent des polymères thermoplastiques choisis dans le groupe constitué par le polyéthylène de faible densité, le polyéthylène linéaire de faible densité, l'alcool polyvinylique, le nylon, le polyester, le polystyrène, le polyméthylpentène, le polyoxy-méthylène, leurs copolymères et leurs mélanges.

5. Pansement adhésif selon la revendication 1, dans lequel la couche intérieure comprend un polymère élastique choisi dans le groupe constitué par un copolymère, catalysé par un métallocène, d'éthylène avec un comonomère choisi dans le groupe constitué par l'octène, l'hexène et le butène ; un monomère d'éthylène propylène diène ; des copolymères de styrène ; un copolymère d'éthylène méthyl acrylate ; des copolymères d'éthylène vinyl acétate et leurs mélanges.

6. Pansement adhésif selon la revendication 1, dans lequel les couches extérieures comprennent un mélange d'un polyéthylène de faible densité et d'un polyéthylène linéaire de faible densité.

7. Pansement adhésif selon la revendication 6, dans lequel la couche intérieure comprend un mélange d'un copolymère de polyéthylène catalysé par un métallocène et d'un copolymère d'éthylène méthyl acrylate.
